# EUROPEAN PATENT SPECIFICATION

(11) **EP 0 565 680 B1**
(45) Date of publication and mention of the grant of the patent: **05.02.1997**
(21) Application number: 92922555.5
(22) Date of filing: 24.10.1992
(51) Int. Cl.: A61F 2/30, A61F 2/36, A61F 2/46

(54) **HIP PROSTHESIS STEM WITH A MEDULLARY CAVITY DRAINAGE SYSTEM**
HÜFTPROTHESENSCHAFT MIT EINEM DRAINAGENSYSTEM FÜR DEN MARKKANAL
TIGE DE PROTHESE DE HANCHE A SYSTEME DE DRAINAGE DE LA CAVITE MEDULLAIRE

(30) Priority: 04.11.1991 DE 4136317
(43) Date of publication of application: 20.10.1993
(73) Proprietor: MERCK PATENT GmbH, D-64271 Darmstadt (DE)
(72) Inventor: SCHMIDT, Joachim, D-5060 Bergisch Gladbach 1 (DE)
(86) International application number: EP9202441
(87) International publication number: WO9308769

(56) References cited:
- EP-A- 0 178 174
- EP-A- 0 220 427
- EP-A- 0 338 981
- EP-A- 0 379 785
- EP-A- 0 434 604
- US-A- 4 994 085

## Description

### BACKGROUND OF THE INVENTION

### Field of the Invention

The invention is related to a hip prosthesis stem which can be cemented in the medullary cavity of the femoral shaft. A hip prosthesis stem is used alone or together with an artificial hip socket (total hip replacement) when disease or injury makes it necessary to replace the head of the femur by an artificial implant.

### Description of Prior Art

The treatment of disorders of the hip joint by total hip replacement has for some years been successful for various indications and is a generally accepted method with about 55,000 implantations per year in Germany. The prosthesis stem can be anchored in the femoral shaft either with or without cement. A large proportion of the prosthesis stems is fixed with bone cement in view of modern cementing techniques and the problems of cementless hip replacement. An overview about recent developments is discussed in detail in WO 88/06 023.

Effective drainage of the medullary cavity or vacuum application of the cement is necessary for modern cementing techniques (WO 88/06 023). A drainage tube (where appropriate combined with a medullary cavity stopper) which is introduced into the medullary cavity from the region of amputation of the femoral head ensures adequate ventilation only on application of the cement. When the prosthesis stem itself is inserted, the drain must be pulled out so early that a pronounced rise in pressure in the medullary cavity results, with embolisation detectable by echocardiography.

This rise in pressure can be avoided by drilling through a separate incision distal of the prosthesis bed in the femoral shaft and, where appropriate, inserting a suction needle. This procedure is described in WO 88/06 023. The disadvantages of this procedure are evident: Additional soft tissue damage and additional bone drilling are necessary and the positioning of the prosthesis is difficult, giving rise to numerous additional complications.

The problem of positioning the prosthesis has been addressed in EP 0 331 623 (and corresponding US 4,919,673). EP 0 331 623 discloses a straight fixing stem for a femoral head prosthesis. The improvement consists of a combination of a centering rod, a medullary cavity stopper, and a borehole along the longitudinal axis of said stem. However the rise in pressure when the prosthesis is inserted cannot be avoided. The invention as disclosed in EP 0 331 623 is restricted to a straight fixing stem. The straight fixing stem is not adapted to the natural curvature of the medullary cavity and causes severely uneven distribution of forces applied to the bone and the prosthesis giving rise to premature material failure and fraction.

In US-A-4,994,085 a femoral prosthesis system is disclosed comprising a stem provided with a longitudinal borehole, a setting guide for alignment of the stem by sliding over the rod of the setting guide while inserting the stem into the medullary cavity, and a plug attached to one end of the rod. However, the setting guide of this prosthesis system is not designed as a drainage tube to which vacuum can be applied.

### BRIEF DESCRIPTION OF THE INVENTION

### Object of the Invention

The disadvantageous rise in pressure in the medullary cavity or the necessity for additional surgical measures with their possible complications can be eliminated according to the invention by providing the hip prosthesis stem with a central borehole in the direction of the stem axis so that, on implantation, it can be pushed over a drainage tube which is fixed centrally in a medullary cavity stopper and has adequate dimensions. By using the drainage tube as a guide the prosthesis can be positioned exactly.

Another object of the invention is to avoid uneven distribution of forces by providing a prosthesis with a curvilinear stem, which follows the natural curvature of the medullary cavity, and with a (curved) central borehole, which facilitates the positioning of the prosthesis.

The present invention thus relates to a prosthetic device for hip joint repair or replacement comprising a femoral prosthesis for implantation into the femoral bone, the stem of said prosthesis being provided with a central borehole in its longitudinal direction, a setting guide fitting slidably into said central borehole, and a medullary cavity stopper fitting into the lower part of the medullary cavity, characterised in that
a) the core rod of the setting guide is designed as a drainage tube to which vacuum can be applied,
b) the medullary cavity stopper is porous allowing the vacuum to act through said porous medullary cavity stopper,
c) there is a detachable fastening means between said drainage tube and said medullary cavity stopper allowing to fasten the distal end of the drainage tube to the central portion of the medullary cavity stopper.

The invention further relates to a prosthesis for repair or replacement of a femoral head by implantation into the femoral bone, characterised in that the prosthesis is provided with a curvilinear stem, following the natural curvature of the medullary cavity, and with a central borehole, which follows the curvature of the stem. Another aspect of the invention is to provide a drainage tube equipped with a fastening means at one end for fastening a porous medullary cavity stopper. Yet another aspect of the invention ist to provide a porous medullary cavity stopper equipped with a structure suitable to be detacheable connected to said fastening means of said drainage tube.

### BRIEF DESCRIPTION OF THE DRAWINGS

In order to simplify the drawings the stem and the central borehole of the prosthesis are depicted based on a design with a straight stem. In preferred embodiments of the invention the stem is formed anatomically to follow the natural curvature of the medullary cavity. Consequently, the central borehole follows this curvature.
- Fig. 1: shows the stem of the prosthesis with its central borehole.
- Fig. 2: shows the porous medullary cavity stopper attached to the drainage tube and a guide sleeve with handle slipped over it.
- Fig. 3: shows the upper part of the femur with the porous medullary cavity stopper and the drainage tube in place, the bone cement filled into the proximal part of the femoral cavity, and the stem of the prosthesis slipped over the drainage tube.

### DETAILED DESCRIPTION OF THE INVENTION

The stem of the femoral prosthesis is made from materials known in prior art for this purpose, e.g. metals or metal alloys. Its size, external shape, the design of the head-neck region, and the shape of the cone are also known in prior art. So are processes for molding the stem of the femoral prosthesis, e.g. by forging, casting, cutting, or welding of subparts, or by combinations of these procedures.

Porous medullary cavity stoppers, their shape, and materials usable for making same are known in prior art. Some of these materials are resorbable in the body, others are not resorbable. Preferably the distal surface and/or the outer surface of the medullary cavity stopper are less porous or unporous. This measure prevents that tissue from the distal femoral cavity is sucked into the stopper. In another preferred embodiment the surface of the medullary cavity stopper facing the proximal femoral cavity is covered with a membrane or fabric unpermeable for the bone cement, but permeable for gases and liquids.

The drainage tube and the guide sleeve are made from materials known for similar purposes, e.g. metals or alloys, polymers or polymer coated metals. Preferred for this purpose is the use of medical tool steel. In the area where the drainage tube and the porous medullary cavity stopper are connected, peripheral holes or slots can be applied to the drainage tube, thus connecting the inner of the drainage tube with the pores of the stopper, in order to improve the effect of the vacuum.

Detacheable fastening means to connect and disconnect the drainage tube with the porous medullary cavity stopper and the guide sleeve with the porous medullary cavity stopper are known in prior art. Examples of suitable fastening means are luer locks, screw or clamping connections, or bayonet fittings. Similar detacheable fastening means can be used to connect and disconnect the second end of the drainage tube with the vacuum pump.

### DETAILED DESCRIPTION OF THE DRAWINGS

An exemplary embodiment is depicted in the figures and is described in detail hereinafter:

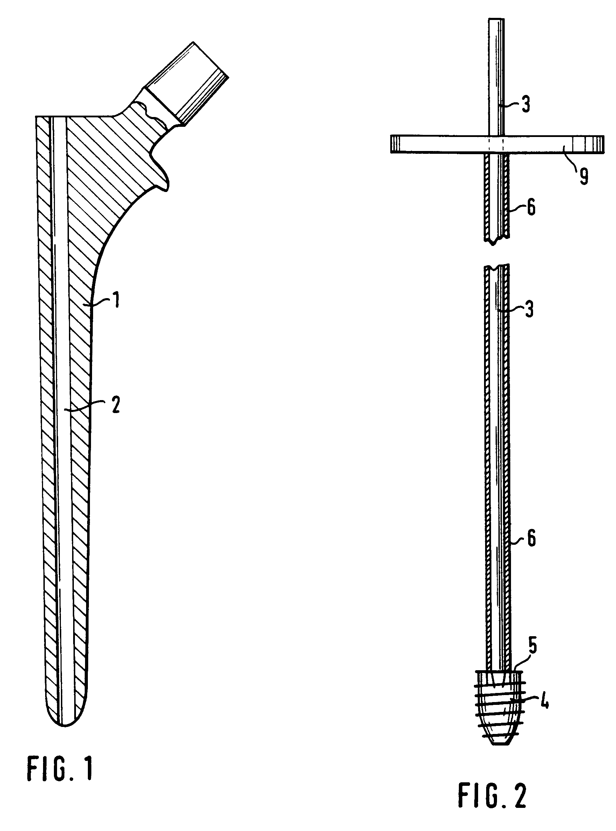

Figure 1 shows a hip prosthesis stem (1) with a size, external shaping, design of the head-neck region and shape of the cone according to the current state of the art. In accordance with Patent Claim 1, this prosthesis stem is provided with a borehole (2) in the direction of the stem axis, which is able to receive slidingly a drainage tube.
Figure 2 shows a drainage tube (3) which is temporarily anchored in a plastic medullary cavity stopper (4). The lower opening of the drainage tube is located beneath a membrane (5) located at the contact surface between the medullary cavity stopper and the cement. This membrane is permeable to liquids and air but not to bone cement. A metal guide sleeve (6) with a handle (9) is pushed over the drainage tube (3) and is anchored with a screw thread in the medullary cavity stopper.

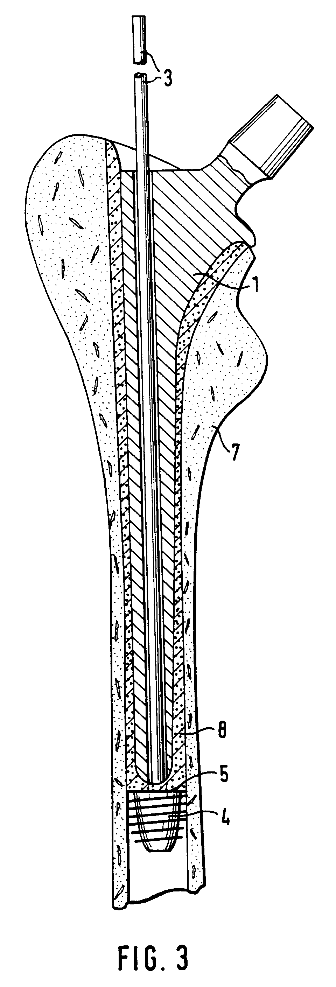

Figure 3 shows a hip prosthesis stem (1) according to Claim 1, which has been implanted in the proximal femoral shaft (7) above a previously positioned drainage tube (3). The medullary cavity stopper (4) prevents spread of the cement socket (8) into the distal medullary cavity. The implantation of the hip prosthesis stem (1) would be complete after removal of the drainage tube (3).

### Surgical procedure

More information about the use of the invention can be gathered from a description of the surgical procedure. Many steps of this procedure are known to the person skilled in the art. The surgical procedure includes the following steps:
a) determination of the medullary cavity diameter with a conventional measuring gauge;
b) selection of the medullary cavity stopper (4) or forming a medullary cavity stopper from a spongiosa block using a diamond hollow cutter,
c) connection of the stopper (4) with the drainage tube (3),
d) fitting of the metal guide sleeve (6) over the drainage tube and anchoring in the medullary cavity stopper (4),
e) positioning of the stopper (4)at the planned depth by means of the marks (10),
f) removal of the guide sleeve (6),
g) preparation of the cement by a conventional technique,
h) application of the cement into the medullary cavity (7), where appropriate, application of a vacuum to the drainage system and sucking in of the cement,
i) implantation of the prosthesis (1) by pushing it with the central borehole (2) over the drainage tube,
j) finally removal of the drainage tube (3).

### Advantages

The advantages which can be achieved with the invention are:
* There is adequate drainage of the medullary cavity throughout the implantation of the prosthesis stem.
* Possibility of application of vacuum to the cement and maintenance of the vacuum on insertion of the prosthesis stem.
* No additional surgical steps with additional possible complications (e.g. separate application of a borehole or of a suction needle on the femoral shaft).
* Reliable central positioning of the prosthesis with reproducibly sufficient cement coating.
* More even distribution of forces along the femur and the stem of the prosthesis.

Without further elaboration, it is believed that one skilled in the art can, using the preceding description, utilize the present invention to its fullest extent. The preferred specific embodiments are, therefore, to construed as merely illustrative, and not limitative of the disclosure in any way whatsoever.

## Claims

1. A prosthetic device for hip joint repair or replacement comprising a femoral prosthesis for implantation into the femoral bone, the stem (1) of said prosthesis being provided with a central borehole (2) in its longitudinal direction, a setting guide (3,6,9) fitting slidably into said central borehole (2), and a medullary cavity stopper (4) fitting into the lower part of the medullary cavity, characterised in that
a) the core rod (3) of the setting guide (6) is designed as a drainage tube to which vacuum can be applied,
b) the medullary cavity stopper (4) is porous allowing the vacuum to act through said porous medullary cavity stopper,
c) there is a detachable fastening means between said drainage tube (3) and said medullary cavity stopper (4) allowing to fasten the distal end of the drainage tube to the central portion of the medullary cavity stopper.

2. The prosthetic device according to claim 1, characterised in that the prosthesis is provided with a curvilinear stem following the natural curvature of the medullary cavity.

3. The prosthetic device according to claim 2, characterised in that the central borehole follows the curvature of the stem.

4. The prosthetic device according to anyone of claims 1 to 3, characterised in that the surface of the porous medullary cavity stopper (4) which is facing the cement bed (8) is equipped with a membrane (5) which is permeable for air and liquids, yet unpermeable for the cement.

5. The prosthetic device according to anyone of claims 1 to 3, characterised in that the drainage tube (3) is equipped with a guide sleeve (6).

6. A prosthetic device according to claim 1, characterised in that the prosthesis is provided with a curvilinear stem following the natural curvature of the medullary cavity, and with a central borehole which follows the curvature of the stem.

## Patentansprüche

1. Prothese für Hüftarthroplastik oder Hüftgelenksersatz, bestehend aus einer zur Implantation in den Oberschenkelknochen bestimmten Kondylarprothese, wobei der Schaft (1) besagter Prothese in seiner Längsrichtung mit einer Mittelbohrung (2) versehen ist, eine Einsatzführung (3, 6, 9) gleitbar in besagte Mittelbohrung (2) paßt, und ein Markhöhlenstopfen (4) in den unteren Teil der Markhöhle paßt, dadurch gekennzeichnet, daß
a) die Kernstange (3) der Einsatzführung (6) als Drainagerohr, an das ein Vakuum angelegt werden kann, ausgebildet ist,
b) der Markhöhlenstopfen (4) porös ist, so daß das Vakuum durch besagten porösen Markhöhlenstopfen hindurch wirken kann,
c) zwischen besagtem Drainagerohr (3) und besagtem Markhöhlenstopfen (4) ein abnehmbares Befestigungsmittel angeordnet ist, wodurch das distale Ende des Drainagerohrs am mittleren Teil des Markhöhlenstopfens befestigt werden kann.

2. Prothese gemäß Anspruch 1, dadurch gekennzeichnet, daß die Prothese einen krummlinigen Schaft aufweist, der der natürlichen Krümmung der Markhöhle folgt.

3. Prothese gemäß Anspruch 2, dadurch gekennzeichnet, daß die Mittelbohrung der Krümmung des Schaftes folgt.

4. Prothese gemäß einem der Ansprüche 1 bis 3, dadurch gekennzeichnet, daß die dem Zementlager (8) zugewandte Oberfläche des porösen Markhöhlenstopfens (4) eine Membran (5) aufweist, die durchlässig für Luft und Flüssigkeiten, aber undurchlässig für Zement ist.

5. Prothese gemäß einem der Ansprüche 1 bis 3, dadurch gekennzeichnet, daß das Drainagerohr (3) mit einer Führungshülse (6) versehen ist.

6. Prothese gemäß Anspruch 1, dadurch gekennzeichnet, daß die Prothese einen der natürlichen Krümmung der Markhöhle folgenden krummlinigen Schaft sowie eine der Krümmung des Schaftes folgende Mittelbohrung aufweist.

## Revendications

1. Dispositif prothétique pour réparation ou remplacement d'une articulation de hanche, comprenant une prothèse fémorale qui est destinée à être implantée dans l'os fémoral, la tige (1) de ladite prothèse étant munie d'un alésage central (2) dans sa direction longitudinale, un guide de pose (3, 6, 9) qui se loge mobile en coulissement dans ledit alésage central (2), et un bouchon de cavité médullaire (4) qui se loge dans la partie inférieure de la cavité médullaire, caractérisé en ce que :
a) la tige d'âme (3) du guide de pose (6) est constituée par un tube de drainage auquel on peut appliquer une dépression,
b) le bouchon de cavité médullaire (4) est poreux, en permettant ainsi au vide d'agir à travers ledit bouchon de cavité médullaire poreux,
c) il est prévu un moyen de fixation détachable entre ledit tube de drainage (3) et ledit bouchon de cavité médullaire (4), ce qui permet de fixer l'extrémité distale du tube de drainage à la portion centrale de le bouchon de cavité médullaire.

2. Dispositif prothétique selon la revendication 1, caractérisé en ce que la prothèse est pourvue d'une tige curviligne qui suit la courbure naturelle de la cavité médullaire.

3. Dispositif prothétique selon la revendication 2, caractérisé en ce que l'alésage central suit la courbure de la tige.

4. Dispositif prothétique selon une quelconque des revendications 1 à 3, caractérisé en ce que la surface du bouchon de cavité médullaire poreux (4) qui est dirigé vers le lit de ciment (8) est équipée d'une membrane (5) qui est perméable à l'air et aux liquides mais imperméable au ciment.

5. Dispositif prothétique selon une quelconque des revendications 1 à 3, caractérisé en ce que le tube de drainage (3) est équipé d'un manchon guide (6).

6. Dispositif prothétique selon la revendication 1, caractérisé en ce que la prothèse est pourvue d'une tige curviligne qui suit la courbure naturelle de la cavité médullaire et d'un alésage central qui suit la courbure de la tige.
